# EUROPEAN PATENT SPECIFICATION

(11) **EP 0 494 847 B1**
(45) Date of publication and mention of the grant of the patent: **05.04.1995**
(21) Application number: 92830004.5
(22) Date of filing: 03.01.1992
(51) Int. Cl.: A61B 5/14, B01L 3/14

(54) **Perforable safety stopper for test tubes**
Durchstechbarer Verschluss für Proberöhrchen
Bouchon perforable pour tubes à essai

(30) Priority: 10.01.1991 IT FI910007
(43) Date of publication of application: 15.07.1992
(73) Proprietor: DIESSE DIAGNOSTICA SENESE s.r.l., I-20123 Milano (IT)
(72) Inventor: Ricci, Antonio, I-53035 Monteriggioni, Siena (IT); Monciatti, Gabriele, I-53100 Siena (IT)
(74) Representative: Mannucci, Michele

(56) References cited:
- EP-A- 0 427 686
- WO-A-81/01238
- DE-A- 3 404 403
- FR-A- 2 291 107

## Description

The invention relates to evacuated test tube devices fitted with a perforable stopper, for the sampling - by means of vacuum suction - of organic liquids and in particular for taking blood samples by means of double-needle devices, with one needle for insertion into the vein and the other having an elastic slip sheath to cover said needle, said sheath being made to slip back upon insertion of the stoppered test tube into the guide cavity encircling said second needle; in this way, the action of perforating the rubber diaphragm in the test tube stopper puts the vein into communication with the evacuated test tube, so drawing off the desired quantity of biological liquid into the test tube. With this system it is possible to use separate test tubes to take successive biological samples in measured quantities so as to avoid taking excess blood and ensuring there are sufficient samples for the individual analyses which are to be made.

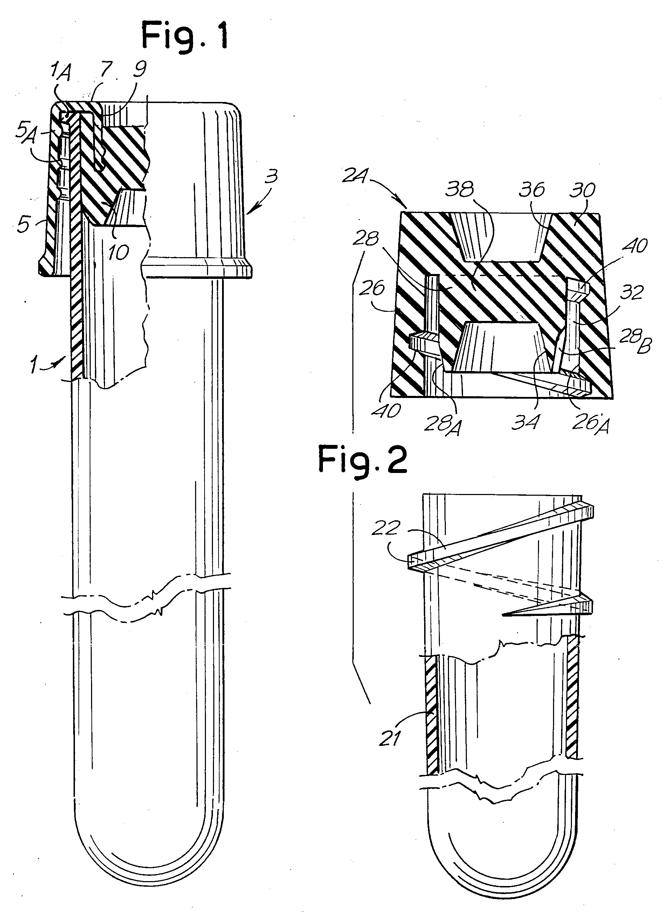

Various kinds of test tubes with perforable stoppers are in use for this purpose. One such test tube is depicted in Fig. 1, where the stopper consists of two coupled components. The test tube 1 ends in an outward rim 1A; the stopper indicated as a whole by 3 has an outer protective mantle 5, an annular connection 7, and an internal tubular tang 9 engaging with a second component 10 which forms the actual stopper creating the seal against the inner wall of the test tube 1; the component 10 is accordingly made of rubber or suchlike material, and engages stably in the part 9 of the component 5 forming the mantle, which latter is generally made of a rigid synthetic plastic. The mantle 5 has a series of internal helical tongues 5A which - as the stopper 3 begins to be withdrawn by a slight axial movement with respect to the test tube 1 - engage over the rim 1A and, through the subsequent rotation of the cap 3, generate a gradual but irregular withdrawing action due to a discontinuous helical coupling action on the part of the tongues 5A. The component 10 forms a diaphragm which is perforable by the sheathed inner needle of the double-needle sampling device.

The invention relates to a perforable stopper by means of which the disadvantages of the system mentioned above and depicted in Fig. 1 are avoided, procuring at the same time greater simplicity in the practical and industrial manufacture of the stopper.

Basically, the perforable stopper - which is removable by rotation after the biological sample has been taken - is made entirely of rubber or suchlike material, with an inner sealing body carrying the perforable diaphragm, an outer mantle to protect against spurts or atomizations of biological fluid when the tube is being opened, and a screwthreaded coupling between the outer wall of the test tube and the inner wall of said mantle so that it can be removed by rotation and inserted by axial force; the cooperating threads can be settled into their proper position by a limited and uniform relative rotation.

In a practical embodiment, the inner surface of the mantle presents the helical channel or channels of the screw thread, and the test tube presents the corresponding male thread or threads.

Advantageously, the surfaces intended to slip over each other may be silicone-treated or otherwise treated to ensure easy sliding and a tight seal. Other surfaces of the cap may also be silicone-treated.

The outer mantle may be extended beyond the sealing body. Externally, the perforable diaphragm is defined by a central depression for preventing the possible escape of a microdrop of sample which may form when the needle is withdrawn.

The invention will be understood more clearly by following the description and the accompanying drawing, which latter shows a practical, non-limiting embodiment of said invention. In the drawing:
Fig. 1 shows the known solution, already described above; and
Fig. 2 shows, in an exploded view, the stopper in section and the open end of the test tube that is to be coupled to said stopper.

As depicted in Fig. 2, the test tube 21, which has the same general shape as known kinds of test tube, has at the open end at least one continuous helical external projection in the form of a screw thread 22, for the purposes indicated below.

In order to seal the test tube 21, which has to be packaged with a certain degree of vacuum inside, a stopper 24 is provided; this is made in a single piece of resilient material (rubber, chlorobutyl, silicone or the like) that exhibits all the properties required for the correct functioning of the system as regards industrial production, as regards the taking of biological samples, and as regards subsequent laboratory operations of opening the tube containing the sample for analysis or test.

As depicted in Fig. 2, the stopper 24 has an outer mantle 26 and an inner sealing body 28 which is joined to the mantle 26 around the extreme part 30 and, with the mantle 26, defines a cleft 32 forming a substantially tubular gap to take the extremity of the wall of the test tube 21. The inner body 28 is slightly frustoconical with a tapered end surface 28A to promote its insertion into the test tube and to obtain a pressure seal against the inner wall of said test tube; this outer surface 28A of the body 28 may comprise at least one recess 28B which serves to create the vacuum in the test tube after the stopper 24 has been deposited and only partly inserted with the body 28 in the opening of the test tube; the recess 28B ensures that a communication passage is left to create the vacuum in the test tube, before proceeding to close the same, by forcing the stopper 24 axially into and against the test tube. In the central part of said stopper, that is in the body 28, an inner recess 34 and a second, outer recess 36 are formed: these define a transverse diaphragm 38 which is perforable by the sheathed inner needle of the double-needle sampling device. The inner surface 26A of the mantle 26 has at least one helical recess 40 forming a negative screw thread, which is cut into the same resilient material (rubber or suchlike) of which the whole stopper is made; the negative screw thread 40 corresponds to the positive screw thread 22 in relief on the outer surface of the test tube 21 adjacent to the opening of the same. The thread or threads 40 (the two cooperating screw threads 22 and 40 may be single- or double-start threads) comes down to the rim of the mantle 26, which extends beyond the body 28, 28A internal to said mantle, for the purposes indicated below.

In the industrial production stage, when the desired degree of vacuum has been reached inside the test tube, with the stopper 24 resting lightly over its opening (the vacuum being transferred from the environment to the inside of the test tube through the recess or recesses 28B and also through the passage that may be offered by the screwthreaded cavity 40), an axial force is applied to the stopper 24 as it rests on the test tube 21, causing the end of the wall of the test tube 21 to enter the gap 32 and closing off the test tube by the forcing of the body 28 into the opening of the tube, while the mantle 26 fits around the outside of the extremity of the test tube. Said operation of fitting the stopper without rotation is possible owing to the elasticity of the screw thread 40 in the stopper. To adjust the relative position of the male thread 22 to the female thread 40, only a limited relative rotation is needed between the test tube 21 and the stopper 24 to bring the relief screw thread 22 into the recess provided for it and represented by the negative thread 40 in the inside of the mantle 26. In this way the test tube containing a calibrated vacuum is ready to take a sample, and this is done by temporarily perforating the diaphragm 38 with the inner needle of the double-needle sampling device. Once the test tube containing the biological sample taken by vacuum suction has been removed, the test tube can be handled easily without particular precautions and can be opened easily by simple rotation and hence unscrewing of the stopper 24. To ensure easy helical sliding when opening the test tube it is advantageous to provide a silicone surface treatment of the rubber at least over the inner wall of the mantle 26 with the thread 40, but also advantageously over the whole stopper 24, the outer surface of which on the mantle 26 may be fluted longi tudinally to provide an easier and safer grip.

The recess 36 enables any microdrop which may form on withdrawal of the needle of the double-needle sampling device to be caught, with no danger of its escaping or of soiling even if the stopper 24 is laid on a work surface. The smoothness with which it is possible to rotate and unscrew the stopper 24, which is due to the continuous screwthreaded coupling 22 and 40, ensures against violent or jerky movements which might cause the, escape of the biological sample in aerosol form or as drops when the test tube is opened in the laboratory for analysis; on the contrary, the gentleness of the un screwing action excludes any danger in this respect.

The making of the stopper 24 in a single piece also gives the ready-to-use test tube long shelf life, with no danger of a decrease in the degree of vacuum in the tube, which can thus be kept for a long period with the confidence that the desired quantity of sample will be taken correctly.

These and other objects and advantages are clear from a reading of the text.

Any reference numbers appearing in the accompanying claims are intended to facilitate the reading of the claims with reference to the description and the drawing, and do not limit the scope of protection represented by the claims.

## Claims

1. A safety stopper (24), perforable with a needle to enable the taking of organic liquids by vacuum into an evacuated test tube (21), this stopper (24) being removable from the test tube (21) by simple rotation, characterized in that it is made entirely of rubber or suchlike material, with an inner sealing body (28) forming the perforable diaphragm (38), an outer mantle (26) for protection and a screw thread (22; 40) for the coupling between the outer wall of the test tube (21) and the inner wall of said mantle (26), so that it can be removed by rotation and inserted by axial force; it being possible to settle the cooperating threads into their proper position by a limited relative rotation.

2. The safety stopper as claimed in the preceding claim, characterized in that there are formed, in the inner surface of the mantle (26), the helical channel or channels (40) of the screw thread, and there are formed on the test tube (21) the corresponding male thread or threads (22).

3. The safety stopper as claimed in the preceding claims, characterized in that the outer mantle (26) extends beyond the sealing body (28) to prevent escapes of the biological sample when the tube is being opened; and externally, the perforable diaphragm (38) is defined by a central depression (36) for preventing the escape of a microdrop which may form when the needle is withdrawn after the sample has been taken.

4. The safety stopper as claimed in the preceding claims, characterized in that at least the surfaces intended to slip over each other are silicone-treated or otherwise treated to ensure easy sliding.

5. The safety stopper, as claimed in the preceding claims, characterized in that the stopper is perforable with the needle of the two-needle devices for the vacuum sampling of biological liquids, made of rubber or the like and capable of being unscrewed.

## Patentansprüche

1. Sicherheitsstopfen (24), durchstechbar mit einer Nadel, um das Aufnehmen organischer Flüssigkeiten durch Vakuum in ein evakuiertes Prüfröhrchen (21) zu ermöglichen, wobei der Stopfen (24) vom Prüfröhrchen (21) durch einfaches Drehen entfernbar ist, **dadurch gekennzeichnet**, daß er vollständig aus Gummi oder dergleichen Material besteht, mit einem das durchstechbare Diaphragma (38) bildenden inneren Dichtkörper (28), einem Außenmantel (26) zum Schutz und einem Schraubengewinde (22; 40) für die Verbindung zwischen der Außenwand des Prüfröhrchens (21) und der Innenwand des Mantels (26), so daß es durch Drehen entfernt und durch axiale Kraft eingesetzt werden kann, so daß es möglich ist, die zusammenwirkenden Gewinde in ihre geeignete Position durch eine begrenzte relative Drehung zu bringen.

2. Sicherheitsstopfen nach dem vorangegangenen Anspruch, **dadurch gekennzeichnet**, daß in der Innenfläche des Mantels (26) der schraubenförmige Gang oder die Gänge (40) des Schraubengewindes ausgebildet sind und daß am Prüfröhrchen (21) der korrespondierende männliche Gewindegang oder die Gewindegänge (22) ausgebildet sind.

3. Sicherheitsstopfen nach den vorangegangenen Ansprüchen, **dadurch gekennzeichnet**, daß sich der Außenmantel (26) über den Dichtkörper (28) hinaus erstreckt, um Austritte biologischer Proben zu verhindern, wenn das Röhrchen geöffnet wird, und daß äußerlich das durchstechbare Diaphragma (38) von einer Vertiefung (36) zum Verhindern des Austritts eines Mikrotropfens begrenzt ist, der sich bilden kann, wenn die Nadel, nachdem die Probe entnommen worden ist, herausgezogen wird.

4. Sicherheitsstopfen nach den vorangegangenen Ansprüchen, **dadurch gekennzeichnet**, daß zumindest die zum Übereinandergleiten bestimmten Flächen silikonbehandelt oder anderweitig behandelt sind, um leichtes Gleiten zu gewährleisten.

5. Sicherheitsstopfen nach den vorangegangenen Ansprüchen, **dadurch gekennzeichnet**, daß der aus Gummi oder dergleichen bestehende und zum Abschrauben geeignete Stopfen mit einer Nadel der Zwei-Nadel-Einrichtungen für die Vakuumprobe der biologischen Flüssigkeiten durchstechbar ist.

## Revendications

1. Bouchon de sécurité perforable (24) comportant une aiguille pour permettre la prise de liquides organiques sous vide dans un tube de test mis sous vide (21), ce bouchon (24) étant amovible du tube de test (21) par simple rotation, caractérisé en ce qu'il est réalisé entièrement en caoutchouc ou en une matière similaire, avec un corps de scellement interne (28) formant la membrane perforable (38), une enveloppe extérieure (26) pour la protection et un filetage (22; 40) destiné au raccordement de la paroi externe du tube de test (21) et de la paroi interne de l'enveloppe (26) de façon à pouvoir l'extraire par rotation et l'introduire sous l'effet d'une force axiale; le filetage coopérant pouvant être ajusté dans la bonne position par une rotation relative limitée.

2. Bouchon de sécurité selon le revendication précédente, caractérisé en ce que dans la surface interne de l'enveloppe (26) sont formées une ou plusieurs gorges hélicoïdales (40) du filetage et en ce que sur le tube de test (21) sont formés le ou les filetages mâles correspondants.

3. Bouchon de sécurité selon les revendications précédentes, caractérisé en ce que l'enveloppe extérieure (26) s'étend au delà des corps de scellement (28) pour empêcher la fuite de l'échantillon biologique au moment où l'on ouvre le tube; et extérieurement, la membrane perforable (38) est définie par une dépression centrale (36) empêchant la fuite d'une microgoutte susceptible de se former au moment où l'aiguille est ôtée lorsque l'échantillon a été prélevé.

4. Bouchon de sécurité selon les revendications précédentes, caractérisé en ce qu'au moins les surfaces prévues pour coulisser l'une sur l'autre sont traitées à la silicone ou traitées d'une autre manière facilitant le glissement.

5. Bouchon de sécurité selon les revendications précédentes, caractérisé en ce que le bouchon est perforable avec l'aiguille des dispositifs à deux aiguilles pour le prélèvement sous vide de liquides biologiques, réalisé en caoutchouc ou analogue, et pouvant être dévissé.
